# EUROPEAN PATENT SPECIFICATION

(11) **EP 1 150 608 B1**
(45) Date of publication and mention of the grant of the patent: **29.12.2004**
(21) Application number: 00908181.1
(22) Date of filing: 11.02.2000
(51) Int. Cl.: A61B 5/117, G06K 9/46

(54) **SYSTEM AND METHOD FOR CHECKING FINGERPRINTS**
SYSTEM UND VERFAHREN ZUR ÜBERPRÜFUNG VON FINGERABDRÜCKEN
SYSTEME ET PROCEDE D'IDENTIFICATION D'EMPREINTES DIGITALES

(30) Priority: 12.02.1999 SE 9900474; 12.02.1999 US 119881 P
(43) Date of publication of application: 07.11.2001
(73) Proprietor: Precise Biometrics AB, 224 64 Lund (SE)
(72) Inventor: ÖBRINK, Marten, S-211 49 Malmö (SE)
(74) Representative: Perklev, Karin Cecilia
(86) International application number: PCT/SE2000/000268
(87) International publication number: WO 2000/049944

(56) References cited:
- EP-A1- 0 813 164
- WO-A1-98/58342
- US-A- 5 610 993

## Description

### Field of the Invention

The present invention relates to a system for checking fingerprints, said system comprising a semiconductor sensor. The invention also concerns a method for checking fingerprints.

### Background Art

It has been known from time immemorial that fingerprints can be used to identify people. Originally a fingerprint was made manually by blackening the fingertip and pressing it against a piece of paper. On the piece of paper a pattern of lines was then left, the lines corresponding to the ridges and the spaces between the lines corresponding to the valleys in the skin of the finger. Nowadays fingerprints are made by means of sensors which sense the pattern in the skin on that part of the finger which is held against the sensor.

Traditionally, the fingerprint technique has been used mostly to identify criminals, but in recent years the technique has also become used for access control.

Prior-art access control systems are based on a person who is to be accepted for access recording a reference fingerprint in safe circumstances. The recorded reference fingerprint is stored in a memory, such as a database, or on a personal data carrier.

When the access control is to be carried out, the person places his finger on a sensor which records a current test fingerprint. The test fingerprint is compared with the previously recorded reference fingerprint/fingerprints stored in the memory to check whether the test fingerprint matches this/one of these and thus belongs to an accepted person. If so, the system signals this. Where appropriate, the signal can control the access to, for instance, a building, a computer or information.

The check whether a test fingerprint matches a previously recorded reference fingerprint is made by means of hardware or software and on the basis of digital representations of the fingerprints. The representation of the fingerprint must be made with high resolution since it is only minor details that distinguish different fingerprints from each other. This means on the one hand that it is a large amount of information that is to be stored for each fingerprint and, on the other hand, that it is a large amount of information that must be compared to establish whether two fingerprints originate from the same person.

The most common method of reducing the amount of information that is stored and compared is to use feature extraction. A complete fingerprint of the person who is to be accepted for access to that protected by the system is recorded, specific features in the fingerprint are identified, such as points in which the ridges divide and points in which the ridges terminate, and the position of these specific features is determined. Merely the information on the position and the categorisation of the specific features are stored in the memory of the system. After this reduction of the amount of information, merely information on the most interesting features in the fingerprint still remains. The features originate from the complete fingerprint.

When the authorisation of a person is to be checked, his complete fingerprint is recorded once more and it is then checked whether the same specific features are to be found in the same places in the test fingerprint as in the previously stored reference fingerprint.

Feature extraction is described in many places in the patent literature. One example is to be found in the introduction to US-5,610,993 which otherwise discloses an alternative method of processing and reducing the amount of information in a fingerprint. More specifically, US-5,610,993 discloses a system in which a complete reference fingerprint and a complete test fingerprint are recorded. Instead of reducing the contents of the fingerprints by determining specific features, this is done by calculating a density value of each pixel and after that carrying out a type of vectorisation of the fingerprint images. When comparing the fingerprints, the images are divided into blocks and a variance calculation is made for each block to determine how well the fingerprints match each other. All blocks must match to a sufficient degree for the fingerprints to be considered to originate from the same person. In the comparison, information from the complete fingerprint is used. If a fingerprint is recorded incompletely, it must be recorded once more.

The sensors used in fingerprint systems are based on various sensing techniques. There are optical sensors which essentially contain a transparent surface, against which the user holds his finger, a lens assembly and the actual sensor which has a light-sensitive surface. The lens assembly projects a reduced image of the fingerprint on the sensor. The optical sensors suffer from the drawback that the recording of the fingerprint does not occur on the scale of 1:1 and that the construction is unwieldy because of the lens assembly. In US-5,610,993 an optical sensor is used.

Semiconductor sensors are also available, where the user's finger is in direct contact with the active surface of the sensor. The currently most common semiconductor sensors are capacitive silicon sensors. They have a sensor surface of at least 100 mm². The capacitive silicon sensors are relatively expensive since they require a large silicon area.

Furthermore, Thomson CSF supply a thermal sensor under the name of FingerChip TM. This sensor has the dimensions 1.5 mm x 14 mm². When a fingerprint is to be read, the user passes his finger over the sensor which at high frequency and by thermal detection records "images" of that part of the finger which at the moment is positioned over the sensor. Subsequently, the "images" are put together to form a single "image" of the complete fingerprint. The complete image is then compared in a conventional manner with a previously recorded image. This sensor requires a smaller silicon surface than the sensors which record a complete fingerprint made by a finger which is held still, but on the other hand this sensor is more difficult to operate since the user must pass his finger over the sensor at a relatively uniform speed for the image to be clear. If the user passes his finger slowly or even makes it stop, the result deteriorates since the sensor is based on differences in temperature that are levelled out immediately if the finger does not move. Besides, new software is necessary to put together the partial fingerprints to form a complete fingerprint. The Thomson fingerprint sensor is also described in EP-0 813 164.

WO 98/58342 discloses a similar system.

### Summary of the Invention

An object of the present invention is to provide a system for checking fingerprints, said system being economical to manufacture and having a sensor which operates in the same way as prior-art sensors for the user.

This object is achieved by a sensor according to claim 1.

A system according to the invention thus comprises a sensor which is adapted to record merely a partial test fingerprint made by a fingertip, and a means for comparing a digital representation of the partial test fingerprint recorded by the sensor with different parts of a digital representation of a prerecorded reference fingerprint which represents a substantially larger finger area than the partial fingerprint.

The prior-art technique has always recorded and processed information from complete fingerprints. A possible reduction of the amount of used information has always been made after the recording of the complete fingerprint, and the saved information has originated from the entire fingerprint area. According to the invention a new line has been taken. The reduction of the amount of information is already carried out in the recording step by choosing to record merely a partial test fingerprint. In the actual check the partial fingerprint is matched with different parts of a reference fingerprint. Thus a partial fingerprint is matched with a substantially larger fingerprint. By utilising almost all the information in the partial test fingerprint, good safety is still obtained.

In this manner, a less expensive system for checking fingerprints can be provided. The sensor will be less expensive since it has a reduced surface and yet the system does not require any software to put together the partial images to form a complete fingerprint. The user does not notice any difference. He holds his finger immovable on the sensor in the same way as on traditional fingerprint sensors.

Preferably the fingerprint comparison is made between a first bitmap representing the partial test fingerprint and a second bitmap representing the reference fingerprint. The comparison can then be made quickly and using a fairly simple algorithm which may consist of, for example, comparing, in different overlap positions between the first and the second bitmap, overlapping parts and giving them different scores depending on whether they are two "ones", two "zeros" or one "one" and one "zero". In this case, bitmap relates to a binarised digital image.

The smaller the surface of the sensor the smaller the silicon cost, and thus, the cost of manufacture. On the other hand, reliability deteriorates the smaller area that is checked. Reliability decreases essentially linearly with the size if the entire partial fingerprint is matched with the reference fingerprint. This means that it is possible to obtain sufficient reliability also by using a smaller sensor, which is not possible if feature matching is used since in that case reliability decreases abruptly if too small a number of specific features is recorded. The choice of a suitable size of the sensor surface therefore is a compromise between price and safety, the intended use being a decisive factor. The surface of the sensor for recording the partial fingerprint is advantageously smaller than 60 mm², preferably smaller than 40 mm² and most preferably smaller than 25 mm².

The sensor is advantageously a semiconductor sensor and preferably a capacitive sensor since it allows recording of fingerprints with high resolution.

The system advantageously comprises a memory for storing one or more reference fingerprints. The memory can be a memory that is fixedly connected to the comparing means or an intelligent card, a smart card, which the user inserts in a reader in the system.

The reference fingerprint can be recorded by a special sensor which is separated from the system described above. In that case the reference fingerprint must be transmitted in a safe manner from the reference fingerprint sensor to the system. However, the system suitably comprises a reference fingerprint sensor for recording reference fingerprints in safe circumstances. The reference fingerprint sensor must be designed so as to record a fingerprint representing a substantially larger finger area than the partial fingerprint. Since as a rule a smaller number of reference fingerprint sensors than the number of test fingerprint sensors is required in a system, it is acceptable for the reference fingerprint sensor to be more expensive.

There have been attempts to tamper with control systems in which use has been made of casts of fingers. Such attempted frauds can be prevented if one lets the sensor record a dynamic fingerprint or, more specifically, a plurality of partial fingerprints from one and the same part of the finger. When the user holds his finger against the sensor, the fingerprint will in fact change depending on how firmly the user presses his finger against the sensor. By first checking that one of the partial fingerprint matches part of a previously stored fingerprint and then checking that there is dynamics in the partial fingerprints, it is possible to ensure that the partial fingerprint originates from a "true" finger of an authorised person. The expression above implying that only a partial fingerprint is recorded thus comprises also the recording of a dynamic partial fingerprint.

A further object of the invention is to provide an alternative method of checking fingerprints, which can be carried out by means of a system which is economical to manufacture and which operates in the same way for the user as prior-art systems. This object is achieved by a method according to claim 7.

According to a second aspect of the invention, this thus concerns a method of checking fingerprints, comprising the steps of recording merely a partial test fingerprint which represents a first finger area; comparing a digital representation of the partial test fingerprint with different parts of a digital representation of at least one prerecorded reference fingerprint representing a second finger area, which is substantially larger than the first finger area, and determining whether the partial test fingerprint originates from the same finger as the prerecorded reference fingerprint on the basis of said comparison.

That stated above regarding the system also concerns the method where applicable.

### Brief Description of the Drawings

The present invention will now be described with reference to the accompanying drawings, in which
Fig. 1 is a schematic view of a first embodiment of a system according to the invention;
Fig. 2 is a schematic view of a second embodiment of a system according to the invention;
Fig. 3 is a schematic view of a third embodiment of a system according to the invention; and
Figs 4 a-c schematically illustrate a partial test fingerprint, a complete reference fingerprint and a comparison of a partial test fingerprint with a reference fingerprint.

### Description of Preferred Embodiments

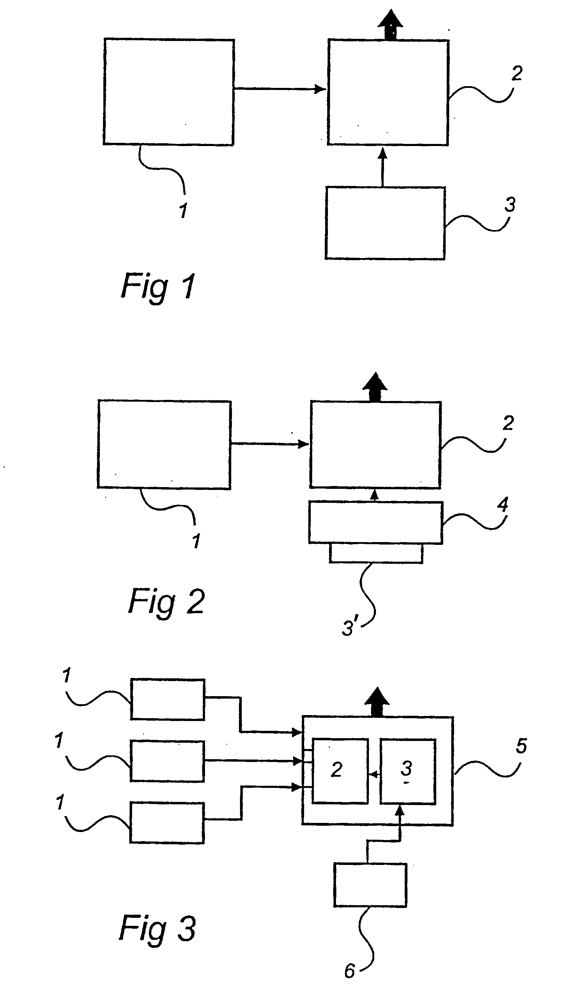

Fig. 1 shows a system for checking fingerprints. The system comprises essentially a sensor 1, comparing means 2 and a memory 3. The sensor 1 is adapted to record a partial test fingerprint made by a finger which is placed on the sensor and feed this to the comparing means 2. The sensor 1 can be, for instance, a capacitive silicon sensor. It can be made up in the same way as commercially available capacitive silicon sensors with the important difference that the surface of the sensor for recording test fingerprints is substantially smaller and so small as to be able to record merely a partial test fingerprint from a normal finger.

The sensor can be of a type which emits an analog output signal or of a type which emits a digital output signal in the form of a grey scale image. In the former case the output signal is A/D converted so as to obtain a digital representation of the fingerprint. In both cases, the digital representation of the fingerprints is preferably thresholded so as to provide a binarised bitmap. The circuits for A/D conversion and thresholding can be integrated with the sensor or be arranged in some other part of the system.

The memory 3 contains one or more reference fingerprints which are recorded in safe circumstances from one or more authorised persons which are entitled to have access to the object or information protected by the system. The reference fingerprints are complete fingerprints from the fingers of the authorised people. They have been recorded either by the same sensor 1 as the partial fingerprints, in which case the user must pass his finger over the sensor and the device must comprise means for putting together the partial fingerprints to form a complete reference fingerprint, or by another sensor that has such a large sensor surface, normally at least 100 mm², as to be able to record a complete fingerprint made by a finger which is kept immovable.

The comparing means 2 are arranged to receive a partial test fingerprint that is to be checked from the sensor 1 and compare this with the reference fingerprint/fingerprints that is/are stored in the memory 3. The comparing means 2 can be implemented in hardware or software and may comprise, for instance, a microprocessor with suitable programs for carrying out the comparison, or consist of a suitably designed ASIC. The output signal from the comparing means somehow indicates whether the partial test fingerprint recorded by the sensor originates from an authorised person whose reference finger-print is stored in the memory. The output signal can be a pure piece of information indicating OK or not OK, or a control signal giving access to an object, for instance, a building or a computer, or to information.

Fig. 1 shows the sensor 1, the comparing means 2 and the memory 3 as separate physical units which are interconnected by means of lines. Other variants are also feasible. The sensor 1 and the comparing means 2 can, for instance, be integrated with each other and be positioned in the same physical unit which is connected to the memory 3, which is positioned in another physical unit. The sensor 1, the comparing means 2 and the memory 3 can also be integrated in a single physical unit. They can also be positioned in different parts of a network and optionally communicate in a wireless manner with each other.

Fig. 2 shows one more alternative, where the memory 3 is a data carrier 3' in the form of a personal card, on which the owner's fingerprint is stored electronically so that it can be read into the comparing means 2 via a reader 4 which in this case is positioned in the same physical unit as the sensor and the comparing unit.

Fig. 3 illustrates a different embodiment of a system, a plurality of sensors 1 being connected to a central unit 5 which contains the memory 3 for storing reference fingerprints and the comparing means 2. The system further comprises a special reference sensor 6 for recording reference fingerprints. The surface of this sensor is considerably larger than the sensor surface of the sensors 1 for recording partial fingerprints.

The system shown in Fig. 3 for checking fingerprints functions as follows. Now assume that the system is used as an access control system in a place of work. Before a user can begin to use the system, his reference fingerprint must be recorded and stored in the memory 3. The recording operation is carried out by means of the reference sensor 6 which records a complete fingerprint made by, for instance, the index finger, binarises and stores it in the form of a bitmap in the memory 3. When the user wants to have access to his place of work, he places his index finger on the sensor 1 which records a partial test fingerprint, which is also binarised to form a bitmap, and transmits it to the comparing unit. The comparing unit collects the bitmap for a first reference fingerprint from the memory 3 and compares this bitmap with the bitmap for the partial test fingerprint. Since the partial fingerprint is much smaller than the reference fingerprint, the partial fingerprint is compared with the reference fingerprint in all conceivable overlap positions. Alternatively, the comparison can be made in selected positions. The comparison can be made by comparing all overlapping pixels and giving different scores when overlapping pixels both are black, both are white or are different. Then all overlapping pixels are checked and the scores are added up. Subsequently, a predetermined criterion, such as a predetermined total of scores, is used to decide whether the images originate from the same finger or not. A more efficient algorithm for matching bitmaps is disclosed in applicant's Swedish Patent Application SE 9704925-8.

If the user's partial fingerprint matches some part of the first reference fingerprint or some part of one of the other reference fingerprints with which the partial fingerprint is compared if there is no match with the first reference fingerprint, the system emits a control signal which opens the lock of the door to the user's place of work. If not, the door remains locked.

The other embodiments described function correspondingly.

It should be pointed out that, of course, the systems can instead be used to lock out unauthorised people, whose fingerprints are stored in the memory 3.

Moreover it is not necessary to compare all the fingerprints in the memory 3 but the user can indicate, by means of a personal code, with which reference fingerprint the comparison is to be made.

Fig. 4a illustrates schematically the partial fingerprint recorded by the sensor 1 and matching only part of the fingertip, which is indicated by the fact that the not recorded part of the pattern is indicated by dashed lines.

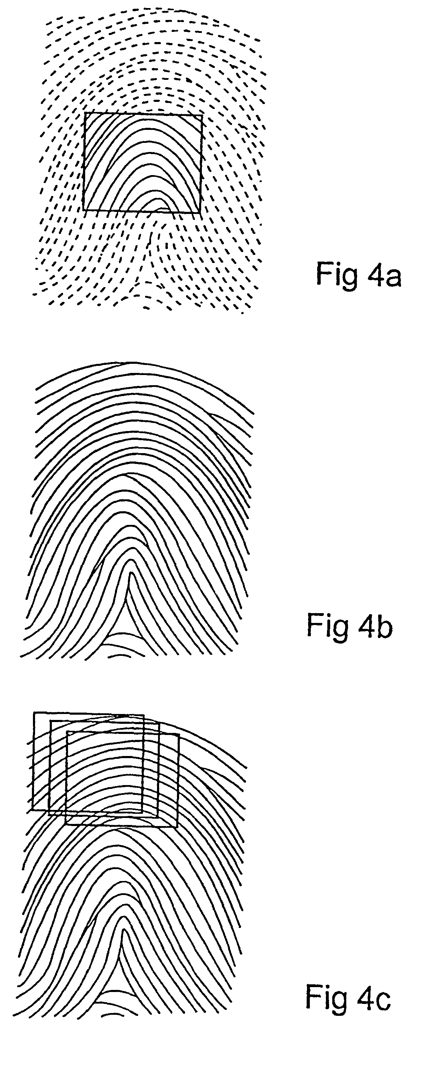

Fig. 4b illustrates schematically the reference fingerprint recorded by the reference sensor 6. As is evident from this Figure, the reference fingerprint matches the entire fingerprint, i.e. the entire portion of the skin of the fingertip which is in contact with a sensor or some other base when the finger is held against this.

Fig. 4c illustrates schematically how the partial fingerprint is compared with different parts of the reference fingerprint. For the sake of clarity, the partial fingerprint is merely shown as a box in a few different positions on the reference fingerprint.

## Claims

1. A system for checking fingerprints, comprising a sensor (1) which is adapted to record merely a partial test fingerprint made by a fingertip; and a means (2) for comparing a digital representation of the partial test fingerprint recorded by the sensor with different parts of a digital representation of a prerecorded reference fingerprint which represents a larger finger area than the partial fingerprint.

2. A system as claimed in claim 1, wherein the comparison is made between a first bitmap representing the partial test fingerprint and a second bitmap representing the reference fingerprint.

3. A system as claimed in claim 1 or 2, wherein the sensor (1) has a surface for recording the partial test fingerprint, said surface being smaller than 60 mm², preferably smaller than 40 mm² and most preferably smaller than 25 mm².

4. A system as claimed in claim 1, 2 or 3, wherein the sensor (1) is a semiconductor sensor, preferably a capacitive sensor.

5. A system as claimed in any one of the preceding claims, further comprising a memory (3, 3') for storing said at least one reference fingerprint.

6. A system as claimed in any one of the preceding claims, further comprising a reference fingerprint sensor (6) for recording said at least one reference fingerprint.

7. A method of checking fingerprints, comprising the steps of
recording merely a partial test fingerprint which represents a first finger area;
comparing a digital representation of the partial test fingerprint with different parts of a digital representation of at least one prerecorded reference fingerprint representing a second finger area, which is larger than the first finger area, and
determining whether the partial test fingerprint originates from the same finger as the prerecorded reference fingerprint on the basis of said comparison.

8. A method as claimed in claim 7, wherein the comparison is carried out between a first bitmap representing the partial test fingerprint and a second bitmap representing the reference fingerprint.

## Patentansprüche

1. System zum Prüfen von Fingerabdrücken, das einen Sensor (1), der so eingerichtet ist, dass er lediglich einen Teil-Test-Fingerabdruck aufzeichnet, der mit einer Fingerspitze gemacht wird, und eine Einrichtung (2) umfasst, die eine digitale Darstellung des Teil-Test-Fingerabdrucks, der von dem Sensor aufgezeichnet wird, mit verschiedenen Teilen einer digitalen Darstellung eines voraufgezeichneten Bezugs-Fingerabdrucks vergleicht, der einen größeren Fingerbereich als der Teil-Fingerabdruck darstellt.

2. System nach Anspruch 1, wobei der Vergleich zwischen einer ersten Bitmap, die den Teif-Test-Fingerabdruck darstellt, und einer zweiten Bitmap, die den Bezugs-Fingerabdruck darstellt, angestellt wird.

3. System nach Anspruch 1 oder 2, wobei der Sensor (1) eine Fläche zum Aufzeichnen des Teil-Test-Fingerabdrucks hat und die Fläche kleiner ist als 60 mm², noch besser kleiner als 40 mm² und am besten kleiner als 25 mm² ist.

4. System nach Anspruch 1, 2 oder 3, wobei der Sensor (1) ein Halbleitersensor, vorzugsweise ein kapazitiver Sensor, ist.

5. System nach einem der vorangehenden Ansprüche, das des Weiteren einen Speicher (3, 3') zum Speichern des wenigstens einen Bezugs-Fingerabdrucks umfasst.

6. System nach einem der vorangehenden Ansprüche, das des Weiteren einen Bezugs-Fingerabdruck-Sensor (6) zum Aufzeichnen des wenigstens einen Bezugs-Fingerabdrucks umfasst.

7. Verfahren zum Prüfen von Fingerabdrücken, das die folgenden Schritte umfasst:
Aufzeichnen lediglich eines Teil-Test-Fingerabdrucks, der einen ersten Fingerbereich darstellt;
Vergleichen einer digitalen Darstellung des Teil-Test-Fingerabdrucks mit verschiedenen Teilen einer digitalen Darstellung wenigstens eines voraufgezeichneten Bezugs-Fingerabdrucks, der einen zweiten Fingerbereich darstellt, der größer ist als der erste Fingerbereich, und
auf Basis des Vergleichs Feststellen, ob der Teil-Test-Fingerabdruck von dem gleichen Finger stammt wie der voraufgezeichnete Bezugs-Fingerabdruck.

8. Verfahren nach Anspruch 7, wobei der Vergleich zwischen einer ersten Bitmap, die den Teil-Test-Fingerabdruck darstellt, und einer zweiten Bitmap, die den Bezugs-Fingerabdruck darstellt, angestellt wird.

## Revendications

1. Système de vérification d'empreintes digitales, comprenant un capteur (1) qui est adapté pour enregistrer seulement une empreinte digitale test partielle faite par le bout d'un doigt ; et un moyen (2) de comparaison d'une représentation numérique de l'empreinte test partielle enregistrée par le capteur avec différentes parties d'une représentation numérique d'une empreinte digitale de référence pré-enregistrée qui représente une zone du doigt plus grande que l'empreinte digitale partielle.

2. Système selon la revendication (1), dans lequel la comparaison est faite entre un premier topogramme binaire représentant l'empreinte test partielle et un second topogramme binaire représentant l'empreinte digitale de référence.

3. Système selon la revendication 1 ou 2, dans lequel le capteur (1) possède une surface destinée à enregistrer l'empreinte digitale test partielle, ladite surface étant inférieure à 60 mm², de préférence inférieure à 40 mm² et de manière préférée entre toutes inférieure à 25 mm².

4. Système selon la revendication 1, 2 ou 3, dans lequel le capteur (1) est un capteur à semi-conducteur, de préférence un capteur capacitif.

5. Système selon l'une quelconque des revendications précédentes, comprenant en outre une mémoire (3, 3') pour stocker ladite au moins une empreinte digitale de référence.

6. Système selon l'une quelconque des revendications précédentes, comprenant en outre un capteur d'empreinte digitale de référence (6) pour enregistrer ladite au moins une empreinte digitale de référence.

7. Procédé de vérification d'empreinte digitale, comprenant les étapes consistant à
enregistrer seulement une empreinte digitale test partielle qui représente une première zone du doigt ;
comparer une représentation numérique de l'empreinte test partielle avec différentes parties d'une représentation numérique d'au moins une empreinte digitale de référence pré-enregistrée qui représente une seconde zone du doigt, qui est plus grande que la première zone du doigt, et
déterminer si l'empreinte digitale test partielle provient du même doigt que l'empreinte digitale de référence pré-enregistrée sur la base de ladite comparaison.

8. Procédé selon la revendication 7, dans lequel la comparaison est réalisée entre un premier topogramme binaire représentant l'empreinte digitale test partielle et un second topogramme binaire représentant l'empreinte digitale de référence.
